# EUROPÄISCHE PATENTSCHRIFT

(11) **EP 1 476 411 B1**
(45) Veröffentlichungstag und Bekanntmachung des Hinweises auf die Patenterteilung: **23.05.2007**
(21) Anmeldenummer: 03706408.6
(22) Anmeldetag: 01.02.2003
(51) Int. Cl.: C07C 17/02, C07C 19/045

(54) **VERFAHREN ZUR HERSTELLUNG VON 1,2-DICHLORETHAN MITTELS DIREKTCHLORIERUNG**
METHOD FOR PRODUCING 1,2-DICHLOROETHANE BY DIRECT CHLORINATION
PROCEDE POUR PRODUIRE DU 1,2-DICHLOROETHANE PAR CHLORATION DIRECTE

(30) Priorität: 21.02.2002 DE 10207217
(43) Veröffentlichungstag der Anmeldung: 17.11.2004
(73) Patentinhaber: Uhde GmbH, 44141 Dortmund (DE); Vinnolit Technologie GmbH & Co. KG, 84508 Burgkirchen (DE)
(72) Erfinder: BENJE, Michael, 64289 Darmstadt (DE); JACULI, Dieter, 84508 Burgkirchen (DE); MIELKE, Ingolf, 84508 Burgkirchen (DE); SCHWARZMAIER, Peter, 84556 Kastl (DE); KREJCI, Klaus, 84489 Burghausen (DE); SCHUBERT, Joachim, 84489 Burghausen (DE); ERTL, Horst, 84568 Pleiskirchen (DE)
(86) Internationale Anmeldenummer: PCT/EP2003/001000
(87) Internationale Veröffentlichungsnummer: WO 2003/070673

(56) Entgegenhaltungen:
- DE-A- 19 910 964
- GB-A- 1 405 926
- US-A- 4 783 564

## Beschreibung

Die Erfindung richtet sich auf ein Verfahren zur Herstellung von 1,2-Dichlorethan, im folgenden als EDC bezeichnet, welches überwiegend als Zwischenprodukt der Herstellung von monomerem Vinylchlorid, im folgenden als VCM bezeichnet, dient, woraus letztlich Polyvinylchlorid, PVC, hergestellt wird. Bei der Umsetzung von EDC zu VCM entsteht Chlorwasserstoff HCl. EDC wird daher bevorzugt aus Ethylen C₂H₄ und Chlor Cl₂ derart hergestellt, dass hinsichtlich des bei den Umsetzungen erzeugten und verbrauchten Chlorwasserstoffes HCl eine ausgewogene Bilanz entsprechend den folgenden Reaktionsgleichungen erreicht wird:

Cl₂ + C₂H₄ → C₂H₄Cl₂ (Rein-EDC) + 180 kJ/Mol (1)

C₂H₄Cl₂ (Spalt-EDC) → C₂H₃Cl (VCM) + HCl - 71 kJ/Mol (2)

C₂H₄ + 2 HCl + 1/₂ O₂ → C₂H₄Cl₂ (Roh-EDC) + H₂O + 238 kJ/Mol (3)

Das Verfahren zur Herstellung von VCM mit ausgewogener HCl-Bilanz, im folgenden kurz "ausgewogenes VCM-Verfahren" genannt, besitzt:
- eine Direktchlorierung, in der aus Ethylen C₂H₄ und Chlor Cl₂ der eine Teil des benötigten EDC in Gegenwart eines Homogenkatalysators erzeugt wird und als sogenanntes Rein-EDC abgegeben wird;
- eine Oxichlorierung, in der aus Ethylen C₂H_{4,} Chlorwasserstoff HCl und Sauerstoff O₂ der andere Teil des EDC erzeugt wird und als sogenanntes Roh-EDC abgegeben wird;
- eine fraktionierende EDC-Reinigung, in der das Roh-EDC zusammen mit dem aus der VCM-Fraktionierung rezirkulierten Rück-EDC und optional zusammen mit dem Rein-EDC von den in der Oxichlorierung und von den in der EDC-Pyrolyse gebildeten Nebenprodukten befreit wird, um ein für den Einsatz in der EDC-Pyrolyse geeignetes, sogenanntes Feed-EDC zu gewinnen, wahlweise kann auch das aus der Direktchlorierung stammende Rein-EDC in der Hochsiederkolonne der EDC-Destillation mitdestilliert werden;
- eine EDC-Pyrolyse, in der das Feed-EDC thermisch gespalten wird; das Spaltgas genannte Reaktoraustrittsgemisch enthält VCM, Chlorwasserstoff HCl und nichtumgesetztes EDC sowie Nebenprodukte;
- eine VCM-Fraktionierung, in der das als Produkt gewünschte Rein-VCM aus dem Spaltgas abgetrennt und die anderen wesentlichen Spaltgasbestandteile Chlorwasserstoff HCl und nichtumgesetztes EDC als Wertstoffe gesondert zurückgewonnen und als wiederverwertbarer Einsatz als Rück-HCl bzw. Rück-EDC im ausgewogenen VCM-Verfahren rezirkuliert werden.

Als Reaktionsmedium in der Direktchlorierung dient bei den meisten im industriellen Maßstab angewandten Verfahren ein umlaufender Strom des Reaktionsprodukts EDC. Dieser kann in einem Schlaufenreaktor mit äußerem oder innerem Umlauf erzeugt werden. Weiterhin kann der Umlaufstrom durch Zwangsumlauf oder Naturumlauf erzeugt werden. Als Katalysator wird vor allem Eisen-III-chlorid verwendet; zusätzlich kann Natriumchlorid, das in der Lage ist, die Bildung von Hochsiedem zu vermindern, als Additiv verwendet werden. Ebenfalls eine inhibierende Wirkung auf die Bildung von Nebenprodukten hat Sauerstoff, der in Chlor, welches in Membran-Elektrolysezellen hergestellt wurde, in geringer Menge (ca. 0.5 -1.5 Vol.%) als Beimischung vorliegt oder als Luft dem Chlor vor Eintritt in den Reaktor zugemischt werden kann.

Der Stand der Technik zur Direktchlorierung wird z.B. in der DE 199 10 964 A1 beschrieben. In dem in DE 199 10 964 A1 beschriebenen Verfahren sollen Nebenreaktionen, vor allem die Weiterchlorierung des EDC zum 1,1,2-Trichlorethan, dadurch unterdrückt werden, dass die Chlorierungsreaktion weitgehend in homogener, flüssiger Phase abläuft. Das in EDC schwerer als Chlor lösliche Ethylen wird im Hauptstrom des umlaufenden Reaktionsmediums EDC in einer Gleichstromblasensäule vollständig aufgelöst. Das in EDC leichter als Ethylen lösliche Chlor wird in einem unterkühlten EDC-Teilstrom aufgelöst und die so erhaltene Lösung von Chlor in EDC dem umlaufenden Hauptstrom, in dem das Ethylen bereits gelöst vorliegt, zugegeben. Die Reaktion (1) in der anschließenden, weitgehend homogenen Reaktionszone verläuft sehr schnell. Es zeigte sich jedoch, das Gasbestandteile (Inerte), die vorwiegend als Beimischungen des Chlors eingetragen wurden, aber auch Sauerstoff aus den Membran-Elektrolysezellen, im Reaktionsraum nicht vollständig in EDC gelöst waren und in Form feiner Gasblasen vorlagen, welche zur Ausbildung einer Phasengrenzfläche führten. Diese Phasengrenzfläche ist offenbar in der Lage, Nebenproduktbildung zu fördern. Als Nebenprodukte wurden vor allem Monochloracetaldehyd, Dichloracetaldehyd und Trichloracetaldehyd (Chloral) beobachtet, welche destillativ nur schwer oder gar nicht von EDC abtrennbar sind und Probleme in nachgeschalteten Prozessstufen verursachen. Ferner wurde die Bildung von 1,1,2-Trichlorethan beobachtet. Der Zusammenhang zwischen der Ausprägung einer Phasengrenzfläche und der Bildung von Nebenprodukten war bisher unbekannt. Im Stand der Technik werden sogar Verfahren beschrieben, die die Ausprägung einer Phasengrenzfläche zwingend erfordern. Hierzu sei auf die US-A 4,783,564 verwiesen, bei sich auch Angaben zur erforderlichen Energiedissipationsdichte finden.

Die Aufgabe der Erfindung besteht daher darin, die Ausbildung einer die Nebenreaktionen fördernden Phasengrenzfläche in der Reaktionszone zu verhindern und so ein wirtschaftliches Verfahren zur Erzeugung von EDC hoher Reinheit zur Verfügung zu stellen.

Diese Aufgabe wird durch das Verfahren nach Patentanspruch 1 gelöst. Die Erfindung löst die Aufgabe, indem
- ein überwiegend Chlor enthaltender Gasstrom in einem Teil des Reaktionsmediums in Lösung gebracht wird, welcher im Wesentlichen frei von gelöstem Ethylen ist,
- die nicht in dieser Lösung gelösten gasförmigen Bestandteile mittels eines Zentrifugalgasabscheiders als Vorrichtung zur Gasabscheidung aus der Lösung entfernt werden, und
- die von ungelösten Gasbestandteilen befreite Lösung mit Ethylen, welches in gelöster Form vorgelegt wird, in Kontakt gebracht wird.

Bezüglich der Reaktionsführung bewirkt diese Vorgehensweise, dass innerhalb der Zone, in der die Hauptreaktion stattfindet, keine Gas-Flüssig-Phasengrenzfläche zur Verfügung steht, die die Bildung von Nebenprodukten, besonders von 1,1,2 - Trichlorethan katalysieren kann. Der auf das Vorhandensein von Rest-Inerten in der Reaktionszone zurückzuführende Anteil an Hochsiedem, insbesondere auch an Sauerstoffverbindungen, wird damit eliminiert, was die Produktqualität verbessert und ein Vorteil der Erfindung ist.

Ein ökonomischer Vorteil ergibt sich auch durch die deutliche Reduktion der Reaktorabgasmenge. Aus Sicherheitsgründen wird die Reaktion meist mit einem Überschuss an Ethylen betrieben. Dieses überschüssige Ethylen findet sich neben dem im Chlor enthaltenen Anteil an Sauerstoff und Inerten nach der Abtrennung des Produktes durch Kondensation im Abgasstrom der Anlage wieder. Um die Bildung eines explosionsfähigen Gasgemischs im Abgassystem zu unterbinden, muss dem Reaktorabgas zur Inertisierung Stickstoff zugegeben werden. Durch die Entfernung der ungelösten Gasbestandteile, insbesondere des darin in der Regel enthaltenen Sauerstoffs, verringert sich der Bedarf der Inertisierung mittels Stickstoff. Das Abgas enthält also nicht nur nicht mehr die ungelösten Gasbestandteile, welche von der Einrichtung zur Gasabscheidung entfernt wurden, sondern auch weniger Inertisierungsgas, weil dieses nicht mehr in diesem Umfang erforderlich ist. Das Abgas wird in einer Anlage zur Durchführung des ausgewogenen Verfahrens zur Herstellung von EDC üblicherweise einer Verbrennungsanlage zugeführt. Da solche Verbrennungsanlagen sich wegen des Einsatzes von Sondermaterialien durch hohe Apparatekosten auszeichnen und die Kapazität dieser Anlagen stark von der Abgasmenge des Direktchlorierungsreaktors abhängt, bedeutet eine Reduktion der Abgasmenge eine erhebliche Kosteneinsparung.

Die Erfindung betrifft auch eine Reihe von Ausgestaltungen, wie mit den in der Vorrichtung zur Gasabscheidung abgeschiedenen gasförmigen Bestandteilen weiter verfahren werden kann. Die folgenden Möglichkeiten können sowohl einzeln als auch parallel zueinander Anwendung finden, wobei der Fachmann je nach den örtlichen Umständen und der Auslastung der Anlage entscheiden wird.

In einer Ausgestaltung des Verfahrens wird wenigstens ein Teil der durch die Vorrichtung zur Gasabscheidung aus der chlorhaltigen Lösung entfernten gasförmigen Bestandteile dem Reaktionsmedium an einer Stelle der Reaktionsstrecke, des Reaktors oder einer Lösevorrichtung zugemischt, an der die das 1,2-Dichlorethan bildende Reaktion von Chlor mit Ethylen bereits weitgehend stattgefunden hat oder nicht mehr stattfinden kann.

In besonders vorteilhafter Weise erfolgt diese Zumischung der durch die Vorrichtung zur Gasabscheidung aus der chlorhaltigen Lösung entfernten gasförmigen Bestandteile in das Reaktionsmedium stromabwärts der Reaktionsstrecke, an der die das 1,2-Dichlorethan bildende Reaktion von Chlor mit Ethylen stattfindet.

Da am Einspeiseort des Sauerstoffs und der Inerte das Ethylen bereits weitgehend abreagiert hat, steht es nicht mehr als Precursor für die Bildung von Sauerstoffverbindungen zur Verfügung. Somit wird auch der Anteil an Sauerstoffverbindungen, für die das Ethylen einen Precursor bildet, eliminiert, was ebenfalls die Produktqualität noch weiter steigert und ein weiterer Vorteil der Erfindung ist.

In einer weiteren Ausgestaltung des Verfahrens wird wenigstens ein Teil der durch die Vorrichtung zur Gasabscheidung aus der chlorhaltigen Lösung entfernten gasförmigen Bestandteile einer Einrichtung zur Nachreaktion zugeführt, wobei diese Einrichtung bei einer niedrigeren Temperatur als die für die Hauptreaktion betrieben wird.

In einer weiteren Ausgestaltung des Verfahrens wird wenigstens ein Teil der durch die Vorrichtung zur Gasabscheidung aus der chlorhaltigen Lösung entfernten gasförmigen Bestandteile einer Einrichtung zur Rückstandsverbrennung ohne weitere Maßnahmen zur Inertisierung zugeführt.

In einer weiteren Ausgestaltung des Verfahrens wird wenigstens ein Teil der durch die Vorrichtung zur Gasabscheidung aus der chlorhaltigen Lösung entfernten gasförmigen Bestandteile einer Einrichtung zur Chlorierung zugeführt.

In besonders vorteilhafter Weise erfolgt diese Zuführung in eine Einrichtung zur Chlorierung, welche dazu dient, Leichtsieder, welche einer Anlage zur Herstellung von VCM aus EDC nach Reaktion (2) entstammen, sogenanntes Rück-EDC, in Hochsieder zu überführen.

Das erfindungsgemäße Verfahren kann mit jeder bekannten Vorrichtung zur Gasabscheidung, welche auch in der Lage ist, feine Bläschen abzuscheiden, betrieben werden. Als besonders vorteilhaft hat sich erwiesen, einen Zentrifugalgasabscheider, wie er auch in der Messtechnik Anwendung findet, zu verwenden.

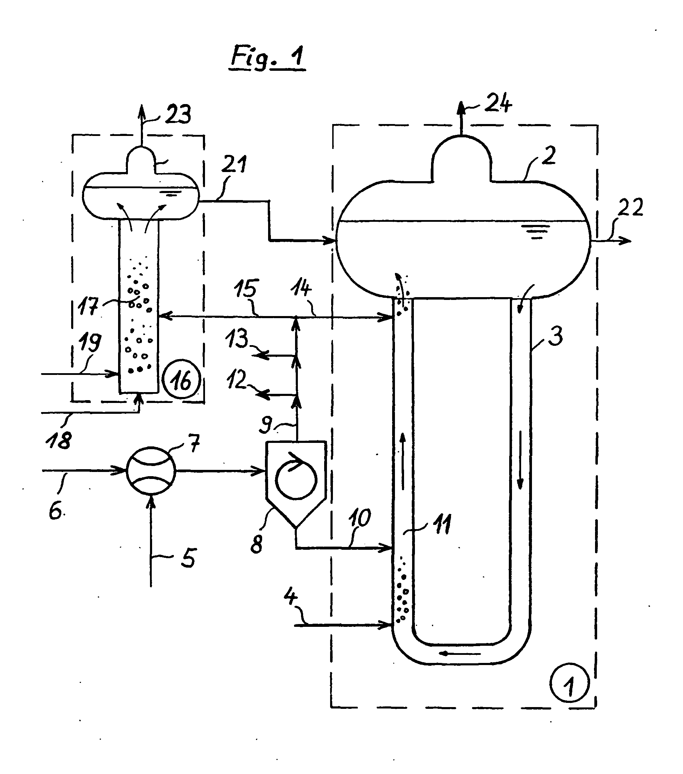

Die Erfindung wird im folgenden anhand eines Beispiels näher erläutert. Fig. 1 zeigt einen Direktchlorierungsreaktor 1, der aus einem Ausgasgefäß 2 und einer Schlaufe 3 besteht, in welcher die Reaktion (1) mit durchgeführt wird, sowie die erfindungsgemäße Gasabscheidung und die erfindungsgemäßen Ausgestaltungen.

Das schwerer als Chlor lösliche Ethylen 4 wird in das in der Schlaufe 3 umlaufende EDC eingebracht und vollständig aufgelöst. Das in EDC leichter lösliche, gasförmige Chlor 5 wird in einem unterkühlten EDC-Teilstrom 6 mittels einer Injektordüse 7 aufgelöst. Der Injektordüse 7 nachgeschaltet wird der Zentrifugalgasabscheider 8, der die nach der Auflösung von Chlor verbleibende, aus Sauerstoff, Inerten und dem partialdruckgemäßen Anteil an Chlor und EDC bestehende Gasphase von der Flüssigphase abtrennt und als Restgas 9 abführt. Die nun homogene Lösung 10 von Chlor in EDC wird der Reaktionszone 11 in der Schlaufe 3 zugeführt, wo die schnelle, homogene Chlorierung des Ethylens zum 1,2-Dichlorethan abläuft.

Das Restgas wird nun auf 4 Teilströme aufgeteilt:
- Der Teilstrom 12 wird einer Einrichtung zur Verbrennung zugeführt,
- der Teilstrom 13 wird einer Chlorierung von Rück-EDC zugeführt,
- der Teilstrom 14 wird dem Direktchlorierungsreaktor 1 zugeführt,und
- der Teilstrom 15 wird einer Einrichtung zur Nachreaktion 16 zugeführt.

Die Einrichtung zur Nachreaktion 16 besteht im Wesentlichen aus einer kleinen Nachreaktionsstrecke 17, die mit unterkühltem, Katalysator enthaltendem EDC 18 betrieben wird, in welches außer dem Teilstrom 15 noch ein kleiner Ethylenstrom 19 gegeben wird. Nach Durchlaufen der Nachreaktionsstrecke 17 und der Ausgasvorrichtung 20 wird der flüssige EDC-Strom 21 in den Direktchlorierungsreaktor 1 geleitet, von wo aus es als Produkt-EDC 22 die Anlage zusammen mit dem dort produzierten EDC verlässt. Das der Ausgasvorrichtung 20 entnommene, nunmehr chlorfreie Abgas 23 wird einer Einrichtung zur Verbrennung zugeführt. Im Unterschied zum EDC-Dampf-Abgasgemisch 24 enthält das chlorfreie Abgas 23 wenig EDC, welches abzuscheiden wäre. Da die Einrichtung zur Nachreaktion 16 hinsichtlich der geringen Einsatzmenge, der niedrigeren Temperatur und der damit verbundenen besseren Löslichkeit von Ethylen, sowie der mit der niedrigen Temperatur weitaus geringeren Neigung der Nebenproduktbildung, unter sehr günstigen Bedingungen betrieben werden kann, lässt sich auch hier eine deutliche Verringerung der Abgasmenge und eine Verbesserung der EDC-Reinheit erreichen.

### Bezugszeichenliste

- 1: Direktchlorierungsreaktor
- 2: Ausgasgefäß
- 3: Schlaufe
- 4: Ethylen
- 5: Chlor
- 6: EDC-Teilstrom
- 7: Injektordüse
- 8: Zentrifugalgasabscheider
- 9: Restgas
- 10: homogene Lösung
- 11: Reaktionszone
- 12: Teilstrom
- 13: Teilstrom
- 14: Teilstrom
- 15: Teilstrom
- 16: Einrichtung zur Nachreaktion
- 17: Nachreaktionsstrecke
- 18: unterkühltes EDC
- 19: Ethylenstrom
- 20: Ausgasvorrichtung
- 21: EDC-Strom
- 22: Produkt-EDC
- 23: chlorfreies Abgas
- 24: EDC-Dampf-Abgasgemisch

## Patentansprüche

1. Verfahren zur Herstellung von 1,2-Dichlorethan in hoher Reinheit unter Einsatz eines im Umlauf geführten flüssigen Reaktionsmediums, welches im Wesentlichen aus 1,2-Dichlorethan und einem Katalysator besteht, wobei dem Reaktionsmedium zumindest Ethylen und Chlor zugeführt werden, **dadurch gekennzeichnet, dass**
• ein überwiegend Chlor enthaltender Gasstrom in einem Teil des Reaktionsmediums in Lösung gebracht wird, welcher im Wesentlichen frei von gelöstem Ethylen ist,
• die nicht in dieser Lösung gelösten gasförmigen Bestandteile mittels eines Zentrifugalgasabscheiders als Vorrichtung zur Gasabscheidung aus der Lösung entfernt werden, und
• die von ungelösten Gasbestandteilen befreite Lösung mit Ethylen, welches in gelöster Form vorgelegt wird, in Kontakt gebracht wird.

2. Verfahren nach Anspruch 1, **dadurch gekennzeichnet, dass** wenigstens ein Teil der durch die Vorrichtung zur Gasabscheidung aus der chlorhaltigen Lösung entfernten gasförmigen Bestandteile dem Reaktionsmedium an einer Stelle der Reaktionsstrecke, des Reaktors oder einer Lösevorrichtung zugemischt wird, an der die das 1,2-Dichlorethan bildende Reaktion von Chlor mit Ethylen bereits weitgehend stattgefunden hat oder nicht mehr stattfinden kann.

3. Verfahren nach Anspruch 2, **dadurch gekennzeichnet, dass** die Zumischung der durch die Vorrichtung zur Gasabscheidung aus der chlorhaltigen Lösung entfernten gasförmigen Bestandteile in das Reaktionsmedium stromabwärts der Reaktionsstrecke, an der die das 1,2-Dichlorethan bildende Reaktion von Chlor mit Ethylen stattfindet, erfolgt.

4. Verfahren nach Anspruch 1, **dadurch gekennzeichnet, dass** wenigstens ein Teil der durch die Vorrichtung zur Gasabscheidung aus der chlorhaltigen Lösung entfernten gasförmigen Bestandteile einer Einrichtung zur Nachreaktion zugeführt wird, wobei diese Einrichtung bei einer niedrigeren Temperatur als die für die Hauptreaktion betrieben wird.

5. Verfahren nach Anspruch 1, **dadurch gekennzeichnet, dass** wenigstens ein Teil der durch die Vorrichtung zur Gasabscheidung aus der chlorhaltigen Lösung entfernten gasförmigen Bestandteile einer Einrichtung zur Rückstandsverbrennung ohne weitere Maßnahmen zur Inertisierung zugeführt wird.

6. Verfahren nach Anspruch 1, **dadurch gekennzeichnet, dass** wenigstens ein Teil der durch die Vorrichtung zur Gasabscheidung aus der chlorhaltigen Lösung entfernten gasförmigen Bestandteile einer Einrichtung zur Chlorierung zugeführt wird.

7. Verfahren nach Anspruch 6, **dadurch gekennzeichnet, dass** die Einrichtung zur Chlorierung dazu dient, Leichtsieder, welche einer Anlage zur Herstellung von monomerem Vinylchlorid aus 1,2-Dichlorethan entstammen, in Hochsieder zu überführen.

## Claims

1. Process for the production of high-purity 1,2-dichloroethane using a circulating stream of liquid reaction fluid which mainly consists of 1,2-dichloroethane and a catalyst, in which at least ethylene and chlorine are admixed to the reaction fluid, **characterised in that**
• a gas stream with chlorine as the main constituent is dissolved in a portion of the reaction fluid, which is essentially free of dissolved ethylene,
• the gaseous constituents non-dissolved in this solution, being removed from the said solution by means of a centrifugal gas separator as device for gas separation and
• the solution freed from non-dissolved gas constituents being brought into contact with solute ethylene supplied for this purpose.

2. Process according to claim 1, **characterised in that** at least part of the gaseous constituents that have been removed from the chlorine-containing solution by the gas separator are admixed to the reaction fluid at a point of the reaction section, the reactor or a dissolving device, in which the reaction of chlorine with ethylene for forming 1,2-dichloroethane has almost terminated or can no longer take place.

3. Process according to claim 2, **characterised in that** the gaseous constituents which have been removed from the chlorine-bearing solution by the gas separator are admixed to the reaction fluid downstream of the reaction section in which the chlorine reacts with the ethylene to yield 1,2-dichloroethane.

4. Process according to claim 1, **characterised in that** at least part of the gaseous constituents removed from the chlorine-bearing solution by means of the gas separator are fed to a facility for secondary reaction, this facility being operated at a lower temperature than applied to the main reaction.

5. Process according to claim 1, **characterised in that** at least part of the gaseous constituents removed from the chlorine-bearing solution by means of the gas separator are fed to a facility for the incineration of residues without rendering inert.

6. Process according to claim 1, **characterised in that** at least part of the gaseous constituents removed from the chlorine-bearing solution by means of the gas separator are fed to a chlorination facility.

7. Process according to claim 6, **characterised in that** the chlorination facility serves to convert light ends from a plant for monomer vinyl chloride production from 1,2-dichloroethane to heavy ends.

## Revendications

1. Procédé pour la production de 1,2-dichloroéthane, de haute pureté, à l'aide d'un courant circulé de fluide de réaction, qui consiste principalement de 1,2-dichloroéthane et d'un fluide catalytique et qui est mêlé, au moins, avec de l'éthylène et de chlore,
**caractérisé en ce que**
• un courant de gaz, qui principalement consiste en chlore, est mis en solution dans une partie du fluide de réaction, la dite partie étant essentiellement libre d'éthylène dissous,
• que les composants non-dissous dans cette solution sont éliminés de la dite solution, moyennant un dispositif séparateur de gaz, et
• que la solution libérée des composants non-dissous du gaz est mis au contact de l'éthylène dissous et fait disponible pour cet objectif.

2. Procédé selon la revendication 1,
**caractérisé en ce que**, au moins, une partie des composants gazeux éliminée de la solution avec une teneur en chlore, à l'aide du dispositif séparateur de gaz, est mêlée avec le fluide de réaction dans le secteur de réaction, dans le réacteur ou dans un récipient à solution, et cela à un point précis où la réaction visant à la formation du 1,2-dichloroéthane par la réaction du chlore avec l'éthylène, a été quasi accomplie et ne pourra plus se poursuivre.

3. Procédé selon la revendication 2,
**caractérisé en ce que** le mélange des composants gazeux, qui ont été séparés de la solution avec une teneur en chlore, moyennant un dispositif séparateur, sont mêlés avec le fluide de réaction dans une zone qui se situe en aval du secteur de réaction dans lequel le chlore réagit avec l'éthylène pour la formation de 1,2-dichloroéthane.

4. Procédé selon la revendication 1,
**caractérisé en ce que**, au moins, une partie des composants gazeux, qui sont séparés de la solution avec une teneur en chlore, moyennant un dispositif séparateur, est amenée vers un dispositif de réaction secondaire, ce dernier fonctionnant à une température inférieure à celle de la réaction primaire.

5. Procédé selon la revendication 1,
**caractérisé en ce que**, au moins, une partie des composants gazeux, qui sont séparés de la solution avec une teneur en chlore, moyennant un dispositif séparateur de gaz, est envoyée vers un dispositif d'incinération de résidus, sans aucun dispositif supplémentaire d'inertisation.

6. Procédé selon la revendication 1,
**caractérisé en ce que**, au moins, une partie des composants gazeux, qui sont séparés de la solution avec une teneur en chlore, moyennant un dispositif séparateur de gaz, est envoyée vers un dispositif de chlorination.

7. Procédé selon la revendication 1,
**caractérisé en ce que** le dispositif de chlorination est utilisé pour faire convertir en une fraction à haut point d'ébullition, les composants à bas point d'ébullition qui sont en provenance d'une usine pour la production de chlorure de vinyl monomère, à partir de 1,2-dichloroéthane.
